# EUROPEAN PATENT APPLICATION

(11) **EP 2 543 330 A1**
(43) Date of publication of application: **09.01.2013**
(21) Application number: 11750410.0
(22) Date of filing: 17.01.2011
(51) Int. Cl.: A61B 19/02

(54) **MEDICAL TRAY**

(30) Priority: 05.03.2010 JP 2010049069
(71) Applicant: Terumo Kabushiki Kaisha, Tokyo 151-0072 (JP)
(72) Inventor: NUMATA, Shigeki, Fujinomiya-shi Shizuoka 418-0015 (JP); YAMAMOTO, Fumio, Fujinomiya-shi Shizuoka 418-0015 (JP); ASAO, Toshihiko, Fujinomiya-shi Shizuoka 418-0015 (JP)
(74) Representative: Dossmann, Gérard
(86) International application number: PCT/JP2011/050645
(87) International publication number: WO 2011/108297

(57) **Abstract**

To provide a medical tray which can be made more compact upon discarding, a medical tray 10 has a bottom portion 11, a side wall 12 provided along an outer periphery of the bottom portion, and a pair of side wall deformation guiding portions 20 provided at opposing positions of the side wall for guiding the side wall when the side wall is deformed into a convex state toward the bottom portion.

## Description

### Technical Field

This invention relates to a medical tray.

### Background Art

In diagnosis, testing and medical treatment in which catheters are used, necessary medical devices such as a catheter and a guide wire, drugs and so forth are set on a medical tray and then taken out, upon treatment, from within the medical tray and used. Then, after the treatment, the medical tray which is not necessitated any more is abandoned into a trash.

The volume of a trash used in a medical field is determined to some degree in accordance with the shape of the inlet of an incineration system, and the cost required for the disposal is in most cases determined in units of trash. Therefore, it is desirable to make the medical tray as compact as possible so that a greater number of medical trays can be placed into the trash. For example, a medical tray disclosed in Patent Document 1 can be made compact upon disposal by folding the same such that a side wall is overlapped with a bottom face.

### Prior Art Document

### Patent Document

Patent Document 1: Japanese Patent Laid-Open No. 2007-75511

### Summary of Invention

However, since a medical tray is generally configured such that a bottom portion thereof is wider than a side wall thereof and the area ratio of the bottom portion occupying in the overall medical tray is high, only if the side wall is folded, the medical tray is liable to be bulky in a trash like the medical tray disclosed in Patent Document 1.

The present invention has been made taking such a subject as described above into consideration, and it is an object of the present invention to provide a medical tray which can be made more compact upon disposal thereof.

A medical tray of the present invention for achieving the object described above has a bottom portion, a side wall provided along an outer periphery of the bottom portion, and a pair of side wall deformation guiding portions provided at opposing positions of the side wall for guiding the side wall when the side wall is deformed into a convex state toward the bottom portion.

Since the medical tray of the present invention configured in such a manner as described above has the side wall deformation guiding portions, when the side wall is deformed into a convex shape toward the bottom portion to fold the bottom portion, force is likely to be applied locally to the bottom portion, and the bottom portion can be folded readily and the medical tray can be made more compact.

If each of the side wall deformation guiding portions includes at least one of a constricted portion formed such that the width of the bottom portion is decreased to the inner side, a side wall grooved portion extending from an upper end of the side wall toward the bottom portion and formed by being recessed to the inner side of the side wall, a side wall upper end recessed portion formed from an upper end of the side wall recessed toward the bottom portion or a side wall upper end stepped portion formed from an upper end of the side wall formed in a stepped state toward the bottom portion, and a flange groove portion formed from a flange formed at an upper end of the side wall and recessed to the inner side of the side wall, then the side wall can be bent locally and deformed readily.

Further, if the medical tray further has a folding guiding portion provided on the bottom portion between the paired side wall deformation guiding portions for guiding the folding of the bottom portion, then the bottom portion is bent at or in the proximity of a line interconnecting the paired side wall deformation guiding portions, and the bottom portion can be folded readily in a desired direction.

If the folding guiding portion includes at least one of a rib or a notch formed on the bottom portion between the paired side wall deformation guiding portions and a bottom edge recessed portion formed from an edge of the bottom portion recessed in a face direction or a bottom edge stepped portion formed from an edge of the bottom portion formed in a stepped state in a face direction, then since bending at a place displaced from a line interconnecting the paired side wall deformation guiding portions or from a portion in the proximity of the line is restricted, or since the bottom portion becomes fragile at or in the proximity of the line, the bottom portion is bent at or in the proximity of the line interconnecting the paired side wall deformation guiding portions and the bottom portion can be folded readily in a desired direction.

If, when the bottom portion is folded, one of opposing portions of the side wall which oppose to each other is placed into the inside of the other of the opposing portions, then the one of the opposing portions of the folded medical tray divided into the two portions is accommodated in the other portion, and the medical tray becomes more compact.

If the bottom portion is divided into two portions by a line interconnecting the paired side wall deformation guiding portions, the portion of the bottom portion on one end side is sized such that the portion is accommodated in the inside of the portion on the other end side, then when the medical tray is folded, the one end side of the folded medical tray divided into the two portions is accommodated in the other end side, and the medical tray becomes more compact.

If, where the bottom portion is divided into two portions by a line interconnecting the paired side wall deformation guiding portions, the portion of the bottom portion on one end side is rounded at corners thereof rather than the portion on the other end side, then the one end side of the medical tray becomes further liable to be accommodated in the other end side.

If the medical tray further has a folding keeping portion for keeping the folding of the bottom portion, then the folded medical tray is less likely to expand into an original state but can keep a compact state.

### Brief Description of the Drawings

[FIG. 1]

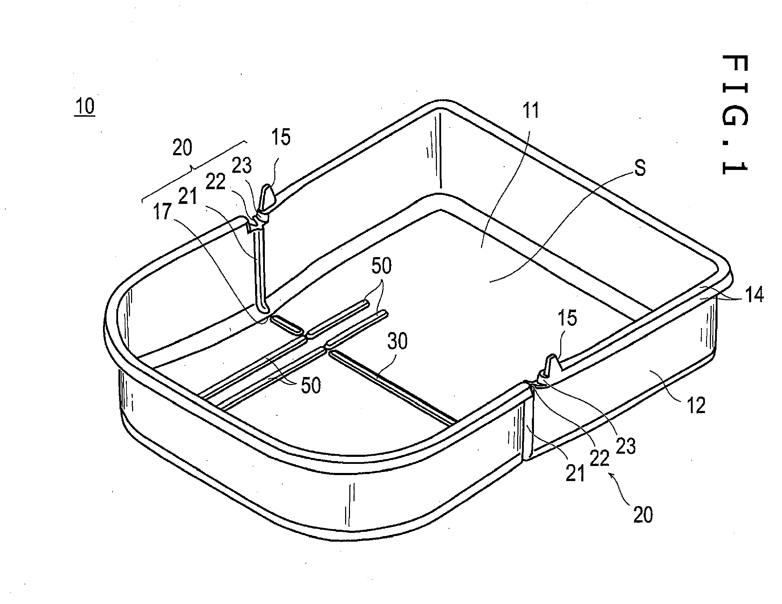

   FIG. 1 is a perspective view showing a medical tray according to an embodiment.
[FIG. 2]

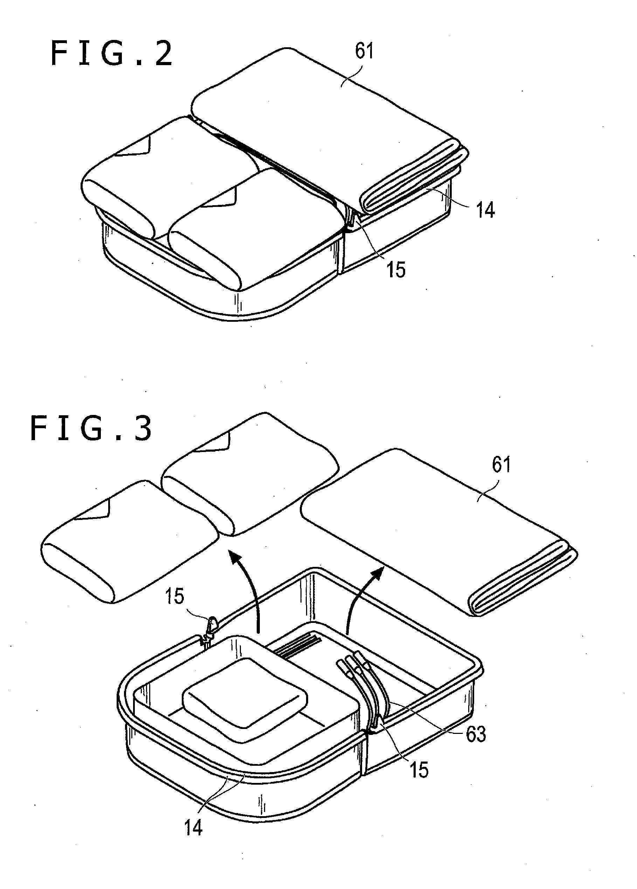

   FIG. 2 is a perspective view showing an example wherein medical devices and so forth are disposed on the medical tray according to the embodiment.
[FIG. 3]
   FIG. 3 is a perspective view showing an example wherein a medical device or the like is taken out from the medical tray according to the embodiment.
[FIG. 4]

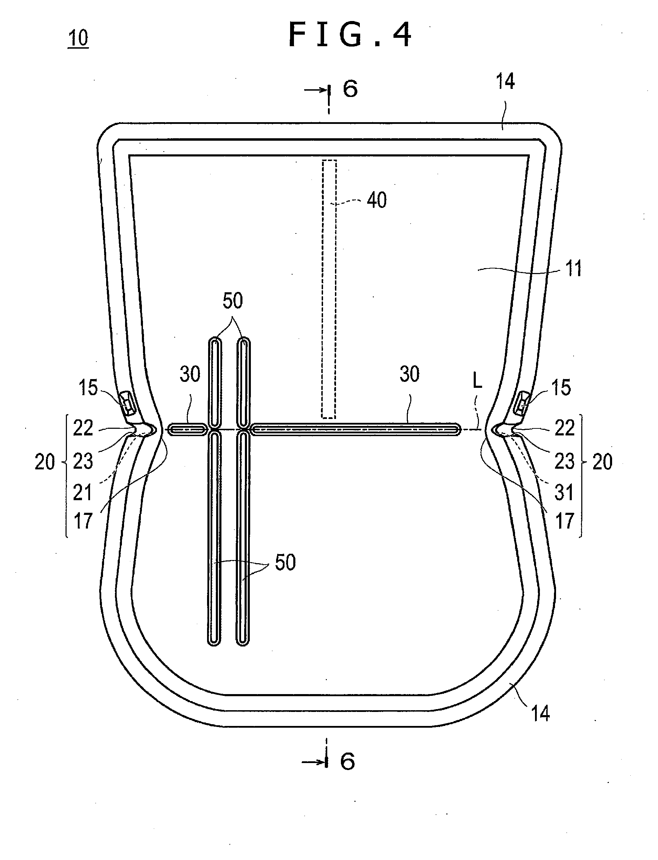

   FIG. 4 is a plan view showing the medical tray according to the embodiment.
[FIG. 5]

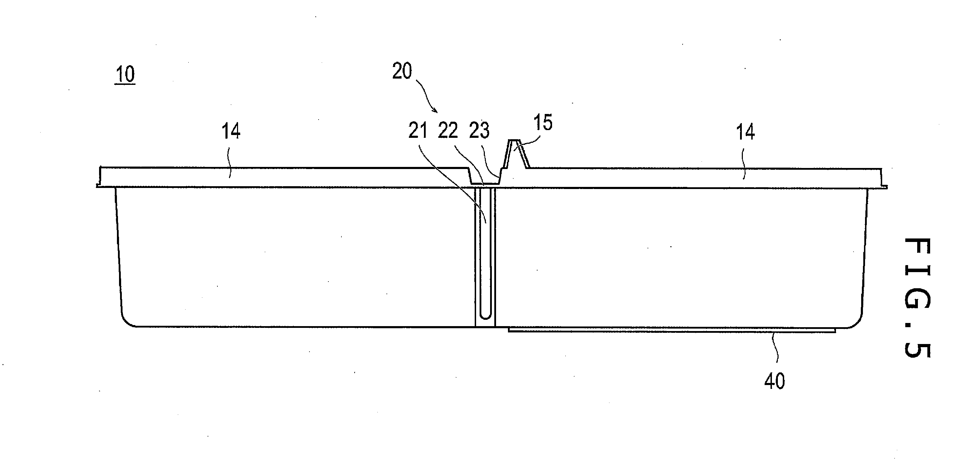

   FIG. 5 is a lateral view showing the medical tray according to the embodiment.
[FIG. 6]

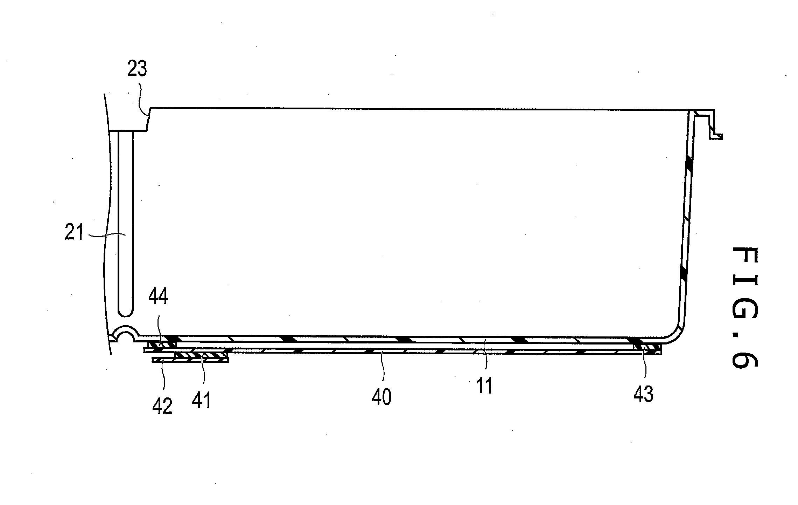

   FIG. 6 is a partial enlarged sectional view showing a cross section taken along a line 6-6 of FIG. 4 in a partially enlarged state.
[FIG. 7]

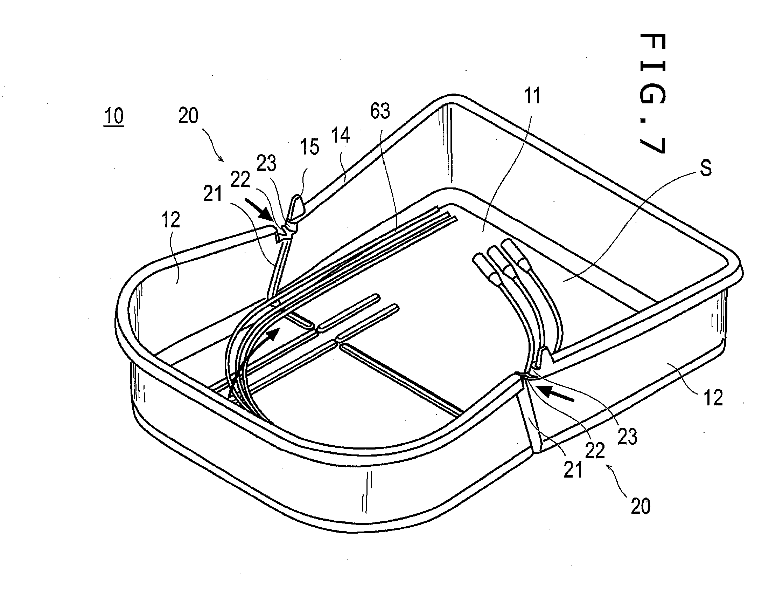

   FIG. 7 is a perspective view showing the medical tray according to the embodiment when it is folded.
[FIG. 8A]

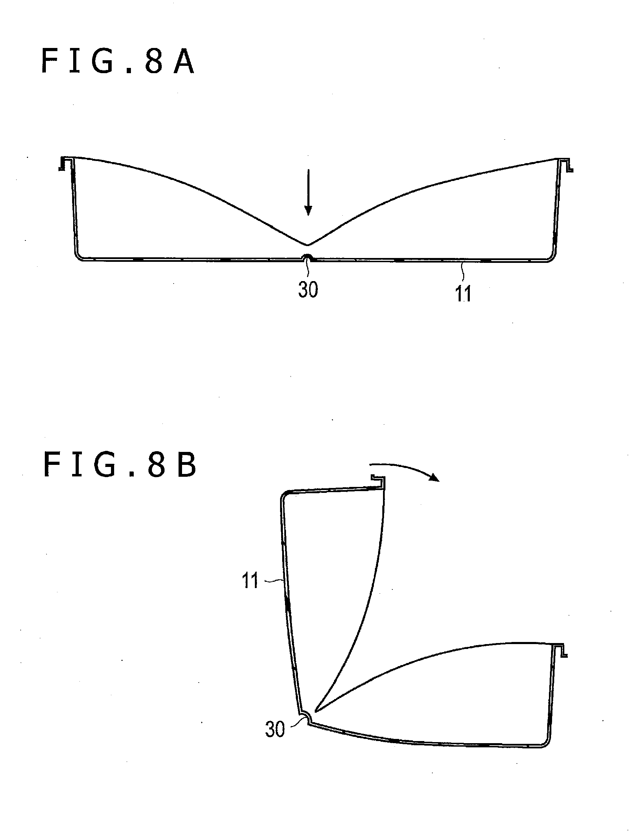

   FIG. 8A is a sectional view schematically illustrating a state in which a side wall is pushed down toward a bottom portion when the medical tray according to the embodiment is folded.
[FIG. 8B]
   FIG. 8B is a sectional view schematically illustrating a state in which the bottom portion is bent when the medical tray according to the present embodiment is folded.
[FIG. 8C]

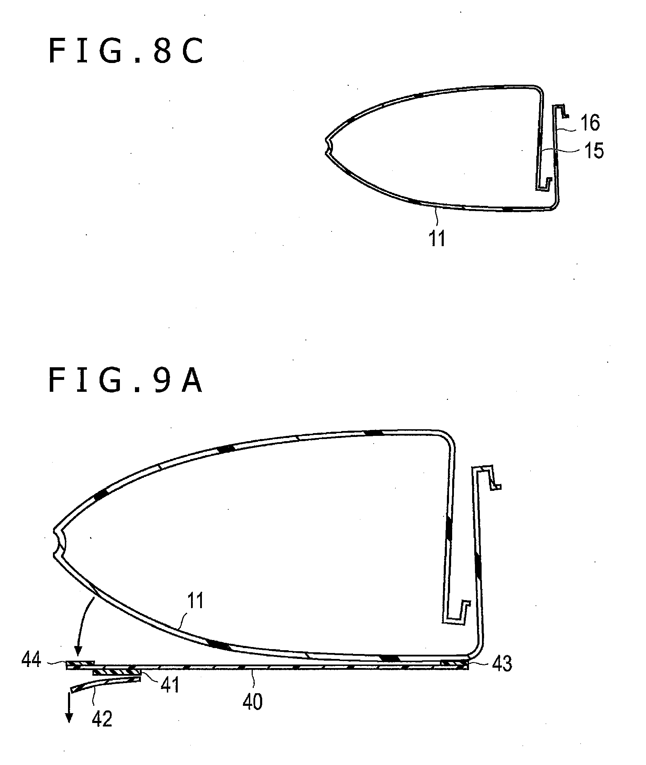

   FIG. 8C is a sectional view schematically illustrating a state wherein one of opposing facing portions of the side wall is pushed into the inner side of the other one of the facing portions when the medical tray according to the present embodiment is folded.
[FIG. 9A]
   FIG. 9A is a sectional view illustrating a state in which one end of a tape temporarily fastened to the bottom portion of the medical tray is exfoliated from the bottom portion after the medical tray is folded.
[FIG. 9B]

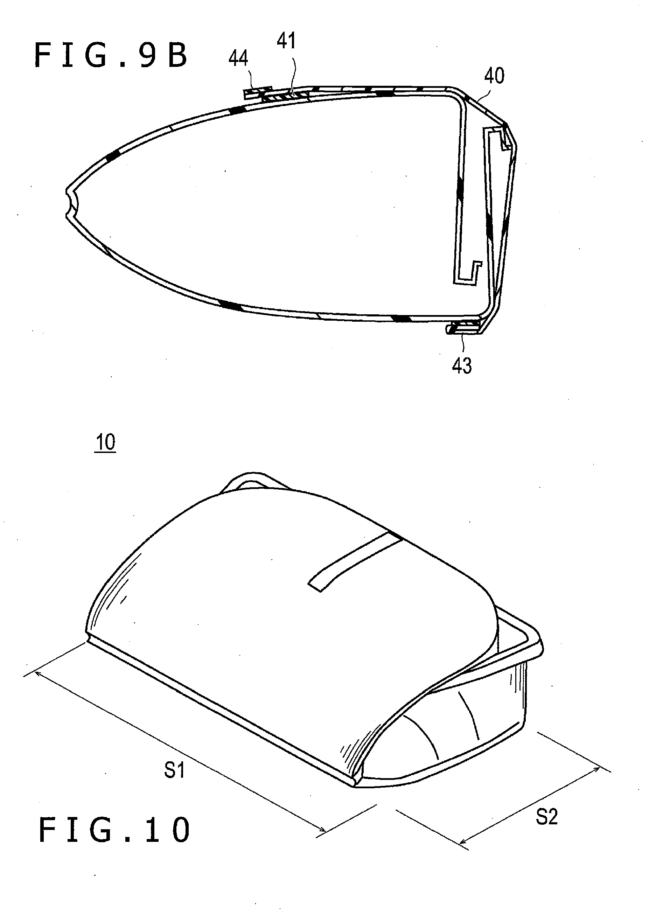

   FIG. 9B is a sectional view of the medical tray whose folded state is kept by a tape.
[FIG. 10]
   FIG. 10 is a perspective view showing the medical tray according to the embodiment after the medical tray is folded.
[FIG. 11]

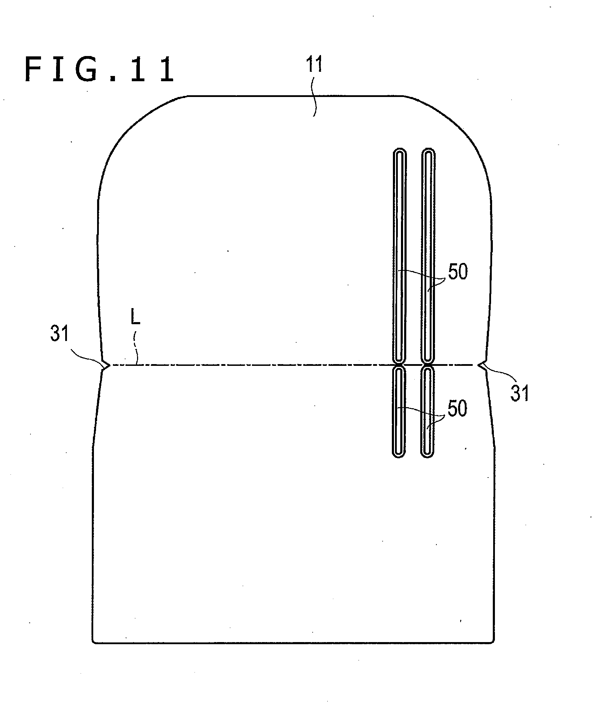

   FIG. 11 is a plan view showing another example of a bottom portion of a medical tray according to the embodiment.
[FIG. 12]

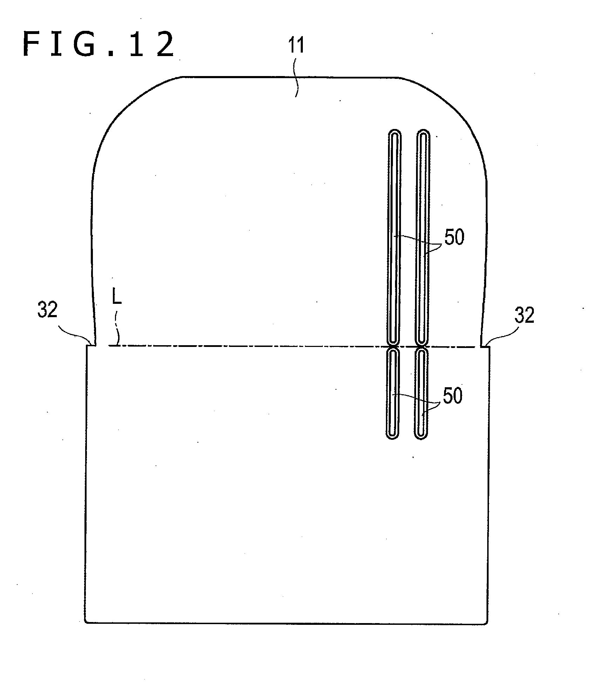

   FIG. 12 is a plan view showing a further example of a bottom portion of a medical tray according to the embodiment.
[FIG. 13]

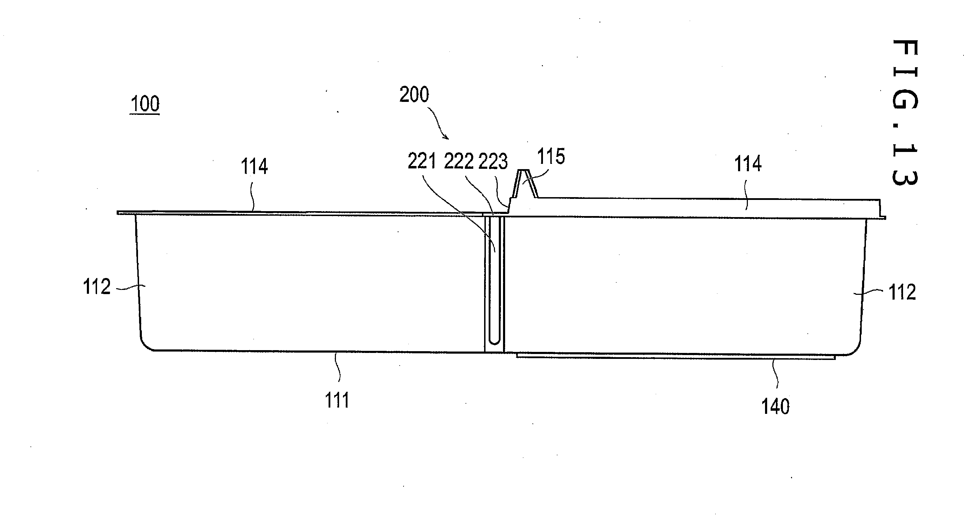

   FIG. 13 is a side elevational view showing a modification to the medical tray according to the embodiment.

### Mode for Carrying Out the Invention

In the following, an embodiment of the present invention is described with reference to the drawings. It is to be noted that the ratio in dimension of the drawings is sometimes exaggerated and is different from an actual ratio for the convenience of description.

As shown in FIG. 1, a medical tray 10 according to the present embodiment includes a bottom portion 11, a side wall 12 provided along an outer periphery of the bottom portion 11, and a flange 14 formed at an upper end of the side wall 12. Further, the medical tray 10 includes a pair of side wall deformation guiding portions 20 provided at opposing positions of the side wall 12, and a rib 30 (folding guiding portion) provided on the bottom portion 11 between the paired side wall deformation guiding portions 20 for guiding the bottom portion 11 upon folding.

The medical tray 10 is made of resin and has a thin wall, and is formed by vacuum forming from a thermoplastic resin sheet of, for example, polyethylene, polypropylene, polystyrene or polyvinylchloride. The bottom portion 11, side wall 12, flange 14, side wall deformation guiding portions 20 and rib 30 are formed integrally.

The medical tray 10 has an accommodating space S formed from the bottom portion 11 and the side wall 12, and the accommodating space S is used to keep medical devices such as catheters, drugs and so forth upon diagnosis, testing and medical treatment. Further, the medical tray 10 can be used also as a packaging tray for packaging a medical device, a drug or the like, and necessary medical devices, drugs and so forth are accommodated in advance in the medical tray 10 and are placed into a packaging back, sterilized and packaged together with the medical tray 10.

Then, as shown in FIGS. 2 and 3, medical instruments, drugs and so forth are placed into a state in which they are set in the medical tray 10 by opening the packaging bag upon carrying out diagnosis, testing and medical treatment. The medical devices may be, in addition to catheters 63 and a drape 61, for example, cotton wools, syringes, guide wires, sheaths, dilators, scissors, tweezers and so forth. The drape 61 is placed on the medical tray 10 and positioned by projections 15 formed on the flange 14.

When treatment is to be carried out, a medical device is taken out from the medical tray 10, or during treatment, a medical device is placed into the medical tray 10 so that it is temporarily stored. Then, after the treatment, any medical device which is not necessitated any more is placed into the medical tray 10 and discarded together with the medical tray 10.

As shown in FIG. 4, constricted portions (reduced width portions) 17 formed such that the width in the proximity of a substantially central portion (in the proximity of a line L) is reduced in comparison with that on the opposite end sides is provided on the bottom portion 11. Since the bottom portion 11 has the constricted portions 17, it is shaped such that edge portions thereof are moderately recessed toward side wall groove portions 21 provided in the proximity of a substantially middle portion of the side wall 12. Further, the bottom portion 11 has a substantially rectangular shape overall, and the constricted portions 17 are provided on the long side. The side wall deformation guiding portions 20 are provided at positions displaced a little from the center of the long side of the bottom portion 11, and a portion on one end side of the bottom portion 11 when it is divided into two portions along the line L interconnecting the paired side wall deformation guiding portions 20 has a size sufficient to allow the same to be accommodated in the inside of the other portion on the other end side and is rounded at corner portions thereof rather than the other end side.

Regarding the side wall 12, since the constricted portions 17 are provided on the bottom portion 11, in the proximity of the line L, the distance between opposing portions of the side wall 12 is smaller than those on the opposite end sides.

The rib 30 projects toward the inner side of the medical tray 10 (toward the accommodating space S side) and extends like a ridge. The rib 30 is a line formed three-dimensionally on the bottom portion 11 and on the line L. On the bottom portion 11, in addition to the rib 30, ridge-like portions 50, which are formed three-dimensionally on the bottom portion 11, are formed. The ridge-like portions 50 project toward the inner side of the medical tray 10 (toward the accommodating space S side) and extend like ridges in order to restrict disposition of an accommodated medical device thereon or to provide strength. In the present embodiment, although the medical tray 10 has the ridge-like portions 50 formed in a direction crossing with the rib 30, where the ridge-like portions 50 cross with the rib 30 in this manner, the ridge-like portions 50 are preferably formed divisionally on the opposite sides across the rib 30 so that folding of the bottom portion 11 may not be obstructed. Further, on the bottom portion 11, an elongated tape 40 (folding keeping portion) for keeping a folded state after the bottom portion 11 is folded is disposed.

As shown in FIGS. 4 and 5, each side wall deformation guiding portion 20 is configured from a constricted portion 17 (refer to FIG. 4) formed such that the width of the bottom portion 11 is reduced to the inner side, a flange groove portion 22 (refer to FIG. 4) formed from the flange 14 recessed to the inner side of the side wall 12, a side wall groove portion 21 (refer to FIG. 5) formed so as to extend from an upper end of the side wall 12 toward the bottom portion 11 and be recessed to the inner side of the side wall 12, and a side wall upper end recessed portion 23 (refer to FIG. 5) formed such that an upper end of the side wall 12 is recessed toward the bottom portion 11.

As shown in FIG. 6, the tape (folding keeping portion) 40 is adhered to the bottom portion 11 by adhesive layers 43 and 44 provided at the opposite ends thereof. The tape 40 is secured at one end thereof by the adhesive layer 43 such that it cannot be spaced away from the bottom portion 11 while it is temporarily fastened at the other end thereof by the adhesive layer 44 such that it can be spaced away from the bottom portion 11. Further, the tape 40 has an adhesive layer 41 provided on a face thereof opposite to the face on which the adhesive layer 44 is disposed. The adhesive layer 41 is covered with a sheet 42 which prevents adhesion.

Now, a folding method of the medical tray 10 is described with reference to FIGS. 7 and 8.

Generally speaking, an operator who carries out folding would push the side wall deformation guiding portions 20 or portions in the proximity of the side wall deformation guiding portions 20 to push down the side wall 12 toward the bottom portion 11 and then fold the bottom portion 11 so that the medical tray 10 is collapsed to substantially one half.

As shown in FIG. 7, for example, when the medical tray 10 is to be discarded together with medical devices such as the catheters 63 after they are used, by sandwiching and pressing the catheters 63 between and by the pushed down side wall 12 and the bottom portion 11, the catheters 63 can be prevented from jumping out by elastic force when the bottom portion 11 is collapsed.

As shown in FIG. 8(A), the crushed side wall 12 is deformed at the side wall deformation guiding portions 20 or in the proximity of the side wall deformation guiding portions 20 into a convex form toward the bottom portion 11. Then, the bottom portion 11 is folded at or in the proximity of the rib 30 as shown in FIG. 8(B) or at or in the proximity of the line L, and one 15 of the opposing face portions of the side wall 12 is placed to the inner side of the other 16 as shown in FIG. 8(C).

Then, the operator would exfoliate the one end of the tape 40 temporarily fastened to the bottom portion 11 from the bottom portion 11 and exfoliate the sheet 42 as shown in FIG. 9(A). Thereafter, the one end of the tape 40 is adhered to one of the two divisional portions of the bottom portion 11 in the folded state by the adhesive layer 41 and keeps the folding of the bottom portion 11 as shown in FIG. 9(B).

Thereafter, the medical tray 10 is discarded in a folded state into a trash as shown in FIG. 10. Although various dimensions of the medical tray 10 can be set suitably, the medical tray 10 preferably has a size with which the medical tray 10 in a folded state can be put as it is into the trash as seen in FIG. 10, and since the opening of a trash used in a medical field frequently has a substantially rectangular shape, the dimension S1 in the longitudinal direction and the dimension S2 in the lateral direction of the bottom portion 11 after it is folded are smaller than the dimensions of the opening of the trash in the longitudinal direction and the lateral direction.

Effects of the present embodiment are described.

In the conventional medical tray described hereinabove, since, different from the present embodiment, the medical tray is folded in such a manner that the face of the side wall is overlapped with the bottom face, the side wall is not deformed in a convex state toward the bottom portion but is deformed in a direction substantially parallel to the face of the bottom face such that it is spaced away from the bottom portion, and the side wall and the bottom portion contact at the faces thereof with each other.

On the other hand, in the present embodiment, since the medical tray 10 has the side wall deformation guiding portions 20, the side wall 12 is deformed in a convex state toward the bottom portion 11, and when the bottom portion 11 is folded, force is likely to be applied locally to the line L or to a portion in the proximity of the line L, and the bottom portion 11 is folded readily and the medical tray 10 can be made more compact.

Further, in the present embodiment, since each side wall deformation guiding portion 20 is configured from the constricted portions 17, flange groove portion 22, side wall groove portion 21 and side wall upper end recessed portion 23, the side wall 12 is bent locally. Consequently, the side wall 12 is likely to be deformed.

Further, in the medical tray 10, since each side wall deformation guiding portion 20 includes the flange groove portion 22, the compatibility of the two conflicting functions of reinforcement of the medical tray 10 by the flange 14 and easiness of folding can be achieved.

Further, with the conventional medical tray described hereinabove, since a waste matter such as cotton wool which is not necessitated any more is hidden by folding the side wall, the side wall must be formed high, and the medical tray becomes deep. Therefore, there is the possibility that the usability may be deteriorated in taking out of a medical device or the like.

On the other hand, in the present embodiment, since a waste matter is concealed by folding the bottom portion 11, the medical tray 10 is not subject to such a structural restriction as in the prior art, and the side wall height can be set freely. Thus, implementation of the medical tray 10 which is superior in usability can be achieved.

Further, in the medical tray 10, since the rib 30 restricts bending at a place displaced from the line L or from a near portion of the line L, the bottom portion 11 is bent at or in the proximity of the line L and is liable to be folded in a desired direction.

Further, with the medical tray 10, since one of the two portions of the bottom portion 11 divided across the line L has a size with which it is accommodated in the inside of the other portion and since, when the bottom portion 11 is folded, the one 15 of the opposing face portions of the side wall 12 which oppose to each other is accommodated in the inside of the other 16, the one of the two divisional portions of the medical tray 10 in the folded state is accommodated in the other divisional portion, and the medical tray 10 becomes more compact.

Further, the tape 40 keeps the folding and the medical tray 10 is less likely to be expanded to its original state, but can keep the compact state.

The present invention is not limited to the embodiment described above but can be modified in various manners within the scope of the claims.

For example, the folding guiding portion is not limited to the rib 30 but may be any element which is formed on the line L and decreases the bending rigidity. Thus, the folding guiding portion may be a notch (line which is formed three-dimensionally) formed such that only one face is recessed in a concave state or may be a bottom edge recessed portion 31 formed such that an edge of the bottom portion 11 is recessed in a face direction or a bottom edge stepped portion 32 formed such that an edge of the bottom portion 11 is formed in a stepped state in a face direction as seen in FIG. 11 or 12. Or, the folding guiding portion may be configured from a combination of one of the rib 30 and a notch and one of the bottom edge recessed portion 31 and the bottom edge stepped portion 32.

Further, the folding guiding portion is not limited to such a linear configuration as the rib 30 in the embodiment described above but may include a bent or curved portion. In other words, the line L interconnects a pair of side wall deformation guiding portions and may be not only a linear line but also a line having a bent or curved portion.

Further, while, in the embodiment described hereinabove, the side wall deformation guiding portion is formed from the constricted portion 17, flange groove portion 22, side wall groove portion 21 and side wall upper end recessed portion 23, the side wall deformation guiding portion is not limited to this but may be configured from only one of them or may be configured from two of them.

Further, while the constricted portion 17 is shaped such that the width of the bottom portion 11 decreases to the inner side from one end side toward a portion in the proximity of a substantially central portion and then increases to the outer side from the portion in the proximity of the substantially central portion toward the other end side, it may otherwise be shaped such that the width of the bottom portion 11 gradually decreases to the inner side from one end side toward a portion in the proximity of a substantially central portion whereas the width of the bottom portion 11 does not vary from the portion in the proximity of the substantially central portion toward the other end side.

Further, the side wall deformation guide portion may include, in place of the side wall upper end recessed portion 23, for example, such a side wall upper end stepped portion 223 formed such that an upper end of a side wall 112 is formed in a stepped state toward a bottom portion 111 as shown in FIG. 13.

Further, the side wall 12 or 112 may be swollen to the outer side from the accommodating space S in the proximity of the bottom portion 11 or 111 so as to form an undercut portion. By providing the undercut portion on the side wall 12 or 112, when an elongated and elastic medical instrument such as a catheter 63 or a guide wire is wound and accommodated into the accommodating space S, the medical instrument can be prevented from jumping out by the elastic force.

Further, the present application is based on Japanese Patent Application No. 2010-049069 filed March 5, 2010, and the disclosed contents thereof are incorporated as a whole herein by reference.

### Description of the Reference Symbols

- 10, 100: Medical tray,
- 11, 111: Bottom portion,
- 12, 112: Side wall,
- 14, 114: Flange,
- 15, 115: Projection,
- 17: Constricted portion
- 20, 200: Side wall deformation guiding portion,
- 21, 221: Side wall groove portion,
- 22, 222: Flange groove portion,
- 23: Side wall upper end recessed portion
- 223: Side wall upper end stepped portion
- 30: Rib (folding guiding portion),
- 31: Bottom edge recessed portion (folding guiding portion),
- 32: Bottom edge stepped portion (folding guiding portion),
- 40: Tape (folding keeping portion),
- 50: Ridge-like portion,
- 61: Drape,
- 63: Catheter,
- L: Line interconnecting side wall deformation guiding portions,
- S: Accommodating space.

## Claims

1. A medical tray, having:
a bottom portion;
a side wall disposed along an outer periphery of the bottom portion; and
a pair of side wall deformation guiding portions provided at opposing positions of the side wall for guiding the side wall when the side wall is deformed into a convex state toward the bottom portion.

2. The medical tray according to claim 1, wherein each of the side wall deformation guiding portions includes at least one of a constricted portion formed such that the width of the bottom portion is decreased to the inner side, a side wall grooved portion extending from an upper end of the side wall toward the bottom portion and formed by being recessed to the inner side of the side wall, a side wall upper end recessed portion formed from an upper end of the side wall recessed toward the bottom portion or a side wall upper end stepped portion formed from an upper end of the side wall formed in a stepped state toward the bottom portion, and a flange groove portion formed from a flange formed at an upper end of the side wall and recessed to the inner side of the side wall.

3. The medical tray according to claim 1 or 2, further having a folding guiding portion provided on the bottom portion between the paired side wall deformation guiding portions for guiding the folding of the bottom portion.

4. The medical tray according to claim 3, wherein the folding guiding portion includes at least one of a rib or a notch formed on the bottom portion between the paired side wall deformation guiding portions and a bottom edge recessed portion formed from an edge of the bottom portion recessed in a face direction or a bottom edge stepped portion formed from an edge of the bottom portion formed in a stepped state in a face direction.

5. The medical tray according to any one of claims 1 to 4, wherein, when the bottom portion is folded, one of opposing portions of the side wall which oppose to each other is placed into the inside of the other of the opposing portions.

6. The medical tray according to claim 5, wherein, where the bottom portion is divided into two portions by a line interconnecting the paired side wall deformation guiding portions, the portion of the bottom portion on one end side is sized such that the portion is accommodated in the inside of the portion on the other end side.

7. The medical tray according to claim 5 or 6, wherein, where the bottom portion is divided into two portions by a line interconnecting the paired side wall deformation guiding portions, the portion of the bottom portion on one end side is rounded at corners thereof rather than the portion on the other end side.

8. The medical tray according to any one of claims 1 to 7, further having a folding keeping portion for keeping the folding of the bottom portion.
